# DEMANDE DE BREVET EUROPEEN

(11) **EP 1 025 837 A1**
(43) Date de publication de la demande: **09.08.2000**
(21) Numéro de dépôt: 00400199.6
(22) Date de dépôt: 26.01.2000
(51) Int. Cl.: A61K 7/48, A61K 7/02

(54) **Utilisation de particules d'un organopolysiloxane hydrophile dans une composition de maquillage ou de soin résistante à l'eau**

(30) Priorité: 08.02.1999 FR 9901447
(71) Demandeur: L'OREAL, 75008 Paris (FR)
(72) Inventeur: Bara, Isabelle, 75013 Paris (FR)
(74) Mandataire: Lhoste, Catherine

(57) **Abrégé**

L'invention se rapporte à l'utilisation de particules d'organopolysiloxane solide élastomérique au moins partiellement réticulé en suspension dans une phase aqueuse, dans une composition à application topique résistante à l'eau. Cette composition est plus spécialement une composition de soin ou de maquillage des lèvres ou une composition de fond teint pour le maquillage aussi bien du visage que du corps humain. Cette composition est douce et fraîche à l'application, s'étale facilement, est non-collante et n'est pas desséchante pour la peau et les lèvres. Elle est parfaitement adaptée aux peaux grasses, du fait de son haut pouvoir matifiant.

## Description

La présente invention se rapporte à une composition de soin et/ou de maquillage de la peau et/ou des lèvres des êtres humains présentant à la fois des propriétés matifiante et de fraîcheur ainsi que de résistance à l'eau, et en particulier à un rouge à lèvres, un eye liner, un fard à joues ou à paupières, ou un fond de teint, ou encore un produit solaire, un déodorant, un shampooing traitant, sous forme de gel aqueux, de lotion, de crème, ou coulé en stick ou en coupelle.

Les compositions de rouge à lèvres et de fond de teint connues comprennent généralement des corps gras tels que des huiles, des composés pâteux et des cires, ainsi qu'une phase particulaire généralement composée de charges et de pigments. Les charges servent généralement à modifier la texture de la composition ainsi qu'à matifier le film ou couche de composition déposé sur la peau et/ou les lèvres alors que les pigments servent généralement à apporter de la couleur à la composition.

L'effet de matité est particulièrement recherché pour les utilisateurs à peaux mixtes ou grasses, ainsi que sous les climats chauds et humides. Les charges matifiantes sont le plus souvent des charges absorbantes comme le talc, la silice, le kaolin ou des charges présentant des propriétés optiques de diffusion de la lumière, propriétés connues sous le nom d'effet "soft focus".

Plus récemment, on a utiliser des polymères matifiants (voir en particulier le document EP-A-790055) tels que des polymères siliconés réticulés connus sous les références commerciales KSG (KSG 6, 16, 17, 18) de la société Shin Etsu, Tréfils de la société Dow Corning ou Gransils pour la société Grand Industrie.

L'inconvénient de ces produits commerciaux est de contenir des huiles de silicones linéaires ou cycliques du type polydiméthylsiloxanes (PDMS) non réticulés et d'apporter un effet huileux, gras, sans effet frais, ce qui ne permet pas ou difficilement leur utilisation en milieu chaud et humide et/ou par les utilisateurs à peaux grasses. De plus ces produits commerciaux, même ceux exempts d'huile de silicone (Tréfils 505 C de Dow Corning par exemple), sont difficilement dispersibles dans un milieu aqueux. Ces produits sont présentés comme des polymères siliconés élastomèriques 〈〈 insolubles dans l'eau 〉〉 (voir notamment le document de Kao EP-A-0855178). Ces polymères, du fait de leur forte incompatibilité avec l'eau, présentent d'excellentes propriétés de résistance ou rémanence à l'eau, ce qui permet de les utiliser dans des compositions dites 〈〈 waterproof 〉〉 notamment de mascara, d'eye liner ou de protection solaire, ces dernières étant très prisées par les consommateurs en vacances en bordure de mer ou de plan d'eau.

Par ailleurs le pouvoir matifiant de ces polymères a tendance à s'estomper au cours du temps, entraînant à nouveau un effet de brillance de la peau, et de gras peu inesthétiques.

Récemment, on a conçu des émulsions contenant ce type de polymère (cf. brevets US-A-5 421 004 de Kose et US-A-5 599 533 d'Estée Lauder) en vue d'améliorer leurs propriétés cosmétiques. Ces émulsions stables, bien qu'apportant moins de gras et plus de fraîcheur que les produits anhydres, perdent leur propriété matifiante initialement apportée par les polymères siliconés réticulés.

Bien qu'il existe des composés de types organosiloxanes réticulés se dispersant en milieu aqueux, comme par exemple, les composés de type KSG 20 ou KSG 21 vendus par la société Shin Etsu, et dont la structure chimique particulière est responsable de cette dispersion en milieu aqueux (présence de groupements polaires leur conférant des propriétés tensioactives), ces composés, contrairement à ceux de la composition de l'invention, n'apportent pas d'effet mat particulier ni de fraîcheur.

Par ailleurs, la société Procter & Gamble a envisagé dans sa demande de brevet WO-A-96/36323 des compositions de mascara de type émulsion eau-dans-huile présentant une longue tenue, une résistance à l'eau et ne laissant pas de traces. Ces compositions contiennent, entre autre, une dispersion aqueuse de polymère, appelée généralement un latex, associée à un tensioactif du type alkyl ou alcoxy diméthicone copolyol, des huiles hydrocarbonées, des pigments et des charges ainsi que des cires. Ces compositions de mascara sont inadaptées à leur utilisation sur la peau. En effet, elle forme sur celle-ci un film continu, après évaporation de l'eau, entraînant des tiraillements et de l'inconfort (dessèchement notamment).

Il subsiste donc le besoin d'une composition résistance à l'eau et matifiante, dont les propriétés persistent sur la peau au cours du temps, et qui apportent en même temps de la fraîcheur et du confort.

De façon surprenante, le demandeur a trouvé que l'introduction de particules d'un organopolysiloxane particulier dans une composition à application topique, et plus spécialement de soin ou de maquillage de la peau ou des lèvres, permettait de remédier aux différents inconvénients ci-dessus et permettait en particulier l'obtention d'un film résistant à l'eau et à propriétés cosmétiques améliorées par rapport à celles des produits résistant à l'eau de l'art antérieur, notamment des propriétés de glissant, de non tiraillement et de non dessèchement des lèvres, ainsi que des propriétés de matifiance et de fraîcheur supérieures à celles des produits matifiants de l'art antérieur.

L'invention s'applique non seulement aux produits de maquillage des lèvres et de la peau des êtres humains mais aussi aux produits de soin et/ou de traitement des lèvres et de la peau humaines. La composition de l'invention peut aussi s'appliquer sur le cuir chevelu.

Ainsi, l'invention a pour objet l'utilisation de particules d'organopolysiloxane solide élastomérique au moins partiellement réticulé en suspension dans une phase aqueuse, dans une composition cosmétique ou pour la fabrication d'une composition à application topique afin d'en augmenter sa résistance à l'eau.

L'invention a encore pour objet l'utilisation de particules d'organopolysiloxane solide élastomérique au moins partiellement réticulé en suspension dans une phase aqueuse dans une composition cosmétique ou pour la fabrication d'une composition à application topique et plus spécialement d'une composition de maquillage ou de soin des matières kératiniques, résistante à l'eau.

L'invention a encore pour objet un procédé pour augmenter la rémanence à l'eau d'une composition cosmétique, consistant à introduire dans la composition des particules d'un organopolysiloxane élastomérique au moins partiellement réticulé en suspension dans une phase aqueuse.

Il est tout à fait surprenant que la composition résiste à l'eau alors qu'elle comprend une phase continue aqueuse. A ce jour, il n'existe pas sur le marché de produit cosmétique résistant à l'eau ayant une phase continue aqueuse.

Par 〈〈 élastomérique 〉〉 on entend un matériau souple, déformable ayant des propriétés viscoélastiques et notamment la consistance d'une éponge ou d'une sphère souple. Son modèle d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Les organopolysiloxanes élastomériques de la composition de l'invention présentent des propriétés de structurant de milieu aqueux et sont aptes à augmenter la viscosité de la phase aqueuse. Ils ne sont pas desséchants pour la peau et apportent de bonnes propriétés cosmétiques, notamment de douceur, de fraîcheur et de matité. Ces nouveaux élastomères conduisent à des compositions confortables à l'application, de bon étalement, douces et non collantes au toucher. Ces propriétés cosmétiques sont dues, d'une part à la texture des organopolysiloxanes et, d'autre part à leurs propriétés comparables à celles de microéponges piégeant les milieux aqueux et en particulier ceux de la composition et ceux dus à la transpiration de la peau. Ils permettent ainsi l'obtention de composition plus ou moins épaissies à bonne rémanence à l'eau.

La composition de l'invention peut se présenter sous forme de pâte, de solide, de crème plus ou moins fluide. Elle peut être une émulsion huile-dans-eau ou eau-dans-huile plus ou moins fluide, un gel hydrophile, solide ou souple. Cette composition peut avoir l'aspect d'une lotion, d'un gel, d'une crème, d'un produit coulé et même se présenter sous forme d'aérosol.

Elle présente, en plus, des avantages ci-dessus, une bonne stabilité.

Les organopolysiloxanes élastomériques conformes à l'invention sont des composés hydrophiles partiellement ou totalement réticulés et de structure tridimensionnelle. L'épaississement de la phase aqueuse par ces élastomères peut être totale ou partielle. Il est tout à fait surprenant que des polymères hydrophiles présentent des propriétés de rémanence à l'eau.

Les élastomères de l'invention se présentent sous forme de poudre ou de gel émulsionné contenant un organopolysiloxane élastomère de structure tridimensionnelle, dispersé dans de l'eau. La dispersion (ou suspension) des particules est homogène.

Les organopolysiloxanes élastomériques selon l'invention peuvent être choisis parmi les polymères réticulés décrits dans la demande JP-A-10/175816. Selon cette demande, ils sont obtenus par réaction d'addition et de réticulation, en présence d'un catalyseur en particulier du type platine, d'au moins :
- (a) un organopolysiloxane (i) ayant au moins deux groupes vinyliques en position α-ω de la chaîne siliconée par molécule ; et
- (b) un organosiloxane (ii) ayant au moins un atome d'hydrogène lié à un atome de silicium par molécule.

En particulier, l'organopolysiloxane (i) est choisi parmi les polydiméthylsiloxanes et est plus spécifiquement un α-ω-diméthylvinyl polydiméthylsiloxane.

Les organopolysiloxanes élastomériques de la composition selon l'invention se présente avantageusement sous forme de suspension aqueuse. Cette suspension peut être notamment obtenu comme suit :
- (a) mélange d'organopolysiloxane (i) et d'organosiloxane (ii) ;
- (b) ajout de la phase aqueuse contenant un émulsifiant au mélange de l'étape (a) ;
- (c) émulsification de la phase aqueuse et dudit mélange ;
- (d) ajout d'eau chaude à l'émulsion de la phase (c) ; et
- (e) polymérisation de l'organopolysiloxane (i) et de l'organosiloxane (ii) en émulsion en présence d'un catalyseur de platine.

L'eau est avantageusement ajoutée à une température de 40-60°C. Après l'étape (e), il est possible de sécher les particules obtenues, pour en évaporer toute ou partie de l'eau piégée.

Les organopolysiloxanes sont sous la forme de particules solides déformables hydrophiles ayant une certaine dureté, mesurable avec un duromètre Shore A (selon la norme ASTM D2240) à la température ambiante ou avec la méthode japonaise JIS-A. Cette dureté peut être mesurée sur un bloc d'élastomère préparé à cet effet comme suit : mélange de l'organopolysiloxane (i) et de l'organosiloxane (ii) ; élimination de l'air du mélange ; moulage et vulcanisation au four à 100°C pendant 30 minutes; refroidissement à température ambiante puis mesure de la dureté. La densité est aussi déterminée sur ce bloc d'élastomère.

En particulier, la dureté Shore est inférieure ou égale à 80 et mieux inférieure à 65. Les organopolysiloxanes de la composition de l'invention sont par exemple ceux commercialisés sous les noms BY 29-122 et BY 29-119 par la société Dow-Corning Electric. On peut aussi utiliser un mélange de ces produits commerciaux. Un bloc d'élastomères selon le produit BY-29122 présente une dureté de 7 et selon le produit BY-29119 une dureté de 30. La densité est de 0,97 à 0,98.

De façon préférentielle, la poudre d'organopolysiloxane élastomérique est présente dans la composition à un taux de 1 à 99% et mieux de 5 à 70%, ce qui correspond à un taux de polymère en matière active de 0,5 à 65% en poids et mieux de 3 à 45%. Elle joue en fait le rôle d'une charge hydrodispersible.

En particulier, les particules d'organopolysiloxane élastomérique (en matière active) ont une taille allant de 0,1 à 500 µm et mieux de 3 à 200 µm. Ces particules peuvent être sphériques, plates ou amorphes avec, de préférence, une forme sphérique.

Ces particules d'organopolysiloxane, pour se disperser de façon stable dans l'eau, peuvent être associées à un ou plusieurs tensioactifs non ioniques, cationiques ou anioniques de HLB ≥ 8.

La proportion de tensioactifs est de préférence de 0,1 à 20 parties en poids pour 100 parties en poids de la composition d'organopolysiloxane élastomérique, et mieux, de 0,5 à 10 parties an poids (cf. description du document JP-A-10/175816).

A ces poudres d'organopolysiloxane élastomérique peuvent être associées des corps gras liquides à température ambiante, appelées huiles, tels que ceux décrits dans le document JP-A-10 /175816, des cires ou des gommes solides à température ambiante, des corps gras pâteux, d'origine animale, végétale, minérale ou synthétique, leurs mélanges ainsi que des poudres inorganiques telles que celles décrites dans ce document.

La phase grasse additionnelle peut être quelconque et contenir des produits fluides à température ambiante comme les huiles siliconées, fluorées, fluorosiliconées, hydrocarbonées éventuellement partiellement siliconées. Ces huiles peuvent être volatiles à température ambiante et pression atmosphérique. Par huile volatile, on entend en particulier une huile susceptible de s'évaporer, en moins d'une heure, au contact de la peau ou des lèvres. Ces huiles peuvent représenter de 0 à 80 % du poids total de la composition.

Comme huiles utilisables dans la composition de l'invention, on peut citer notamment :
- les huiles hydrocarbonées d'origine animale telles que le perhydrosqualène ;
- les huiles hydrocarbonées végétales telles que les triglycérides liquides d'acides gras, par exemple les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel ;
- les huiles de formule R¹COOR² dans laquelle R¹ représente le reste d'un acide gras supérieur comportant de 7 à 19 atomes de carbone et R² représente une chaîne hydrocarbonée ramifiée contenant de 3 à 20 atomes de carbone comme par exemple l'huile de Purcellin ;
- les hydrocarbures linéaires ou ramifiés, d'origine minérale ou synthétique tels que les huiles de paraffine volatiles ou non et leurs dérivés, la vaseline, les polydécènes, le polyisobutène hydrogéné tel que le parléam ;
- les esters et les éthers de synthèse comme le myristate d'isopropyle, des octanoates, décanoates ou ricinoléates d'alcools ou de polyalcools ;
- des alcools gras comme l'octyl dodécanol ou l'alcool oléique ;
- les huiles fluorées partiellement hydrocarbonées et/ou siliconées comme celles décrites dans le document JP-A-2-295912 ;
- les huiles siliconées comme les polyméthylsiloxanes à chaîne siliconée linéaire ou cyclique, liquides ou pâteux à température ambiante, les phényl diméthicones, les phényl triméthicones et les polyméthylphénylsiloxanes ;
- leurs mélanges.

Avantageusement, la composition selon l'invention peut contenir des cires hydrocarbonées, fluorées ou siliconées ou leurs mélanges, qui peuvent être solides ou semi-solides (sous forme d'une pâte) à température ambiante. Ces cires peuvent être d'origine végétale, minérale, animale et/ou synthétique. En particulier, ces cires présentent une température de fusion supérieure à 25 °C et mieux supérieure à 45 °C.

Les cires siliconées peuvent être des cires comportant une structure siliconée et des motifs à une ou plusieurs chaînes alkyle ou alcoxy pendantes et/ou en bout de structure siliconée, ces chaînes étant linéaires ou ramifiées et comportant de 10 à 45 atomes de carbone. Ces cires sont appelées respectivement des alkyldiméthicones et des alcoxydiméthicones. Par ailleurs, ces chaînes alkyle peuvent comporter une ou plusieurs fonctions ester.

Comme autres cires utilisables dans l'invention, on peut citer les cires d'origine animale comme la lanoline, la cire d'abeille ; les cires d'origine végétale telles que la cire de Carnauba ou de Candellila ; les cires d'origine minérale, par exemple de paraffine, de lignite ou les cires microcristallines, la cérésine ou l'ozokérite ; les cires synthétiques comme les cires de polyéthyléne ; leurs mélanges.

Ces corps gras peuvent être choisis de manière variée par l'homme du métier afin de préparer une composition ayant les propriétés, par exemple de consistance ou de texture, souhaitées.

En particulier, la présence de cires permet d'assurer une bonne résistance mécanique, notamment lorsque la composition se présente sous la forme d'un stick.

D'une manière générale, la composition peut comprendre de 0 à 50% du poids total de la composition de cire et de préférence de 10 à 30%.

La composition de l'invention peut comprendre, en outre, au moins un ingrédient additionnel usuellement utilisé dans le domaine concerné choisi parmi les antioxydants, les huiles essentielles, les conservateurs, les actifs cosmétiques ou dermatologiques comme les hydratants (glycérine), les vitamines, les acides gras essentiels et les filtres solaires lipophiles, les polymères liposolubles notamment hydrocarbonés tels que les polyalkylènes, les gélifiants de phase aqueuse, les gélifiants de phase grasse, les parfums, les tensioactifs et leurs mélanges.

Ces ingrédients additionnels peuvent être présents dans la composition selon les quantités habituellement utilisées et par exemple à raison de 0 à 20% du poids total de la composition et mieux de 0,1 à 10%.

Avantageusement, la composition de l'invention contient comme ingrédients additionnels un ou plusieurs gélifiants de phase aqueuse. Parmi les gélifiants de phase aqueuse utilisables selon l'invention, on peut citer : les gélifiants cellulosiques hydrosolubles tels que l'hydroxyéthylcellulose, la méthylcellulose, l'hydroxypropylcellulose et la carboxyméthylcellulose ; la gomme de guar ; la gomme de guar quaternisée ; les gommes de guar non-ioniques comprenant des groupements hydroxyalkyle en C₁-C₆ ; les gommes de xanthane, de caroube, de scléroglucane, de gellane, de rhamsan, de karoya ; les alginates, la maltodextrine, l'amidon et ses dérivés, l'acide hyaluronique et ses sels; les argiles et notamment les montmorillonites, les hectorites ou bentones, les laponites ; les polymères à groupement carboxylique comme les acides polyacryliques réticulés au moins partiellement neutralisé tels que les 〈〈Carbopol〉〉 ou 〈〈Carbomer〉〉 de la Société Goodrich (Carbomer 980 par exemple neutralisé par de la triéthanolamine -TEA en abréviation-) ; les polymères poly(métha)crylates de glycéryle ; la polyvinylpyrrolidone ; l'alcool polyvinylique ; les polymères et les copolymères réticulés d'acrylamide ; les homopolymères réticulés de chlorure de méthacryloyloxyéthyltriméthylammonium; les polyuréthanes associatifs et leurs mélanges.

Selon l'invention, le gélifiant de phase aqueuse est de préférence choisi parmi la gomme de xanthane, les argiles (bentone ou laponite), les polyuréthanes associatifs, les épaississants cellulosiques, notamment l'hydroxyéthyl cellulose, et les acides polyacryliques réticulés au moins partiellement neutralisés.

Bien entendu l'homme du métier veillera à choisir les éventuels ingrédients additionnels complémentaires et/ou leur quantité de telle manière que les propriétés avantageuses de la composition selon l'invention ne soient pas, ou substantiellement pas, altérées par l'adjonction envisagée. En particulier, ces additifs ne devront pas nuire à l'homogénéité, la stabilité, le confort, la matité, la fraîcheur et la résistance à l'eau de la composition.

La composition selon l'invention peut se présenter sous la forme d'un produit coloré et notamment de maquillage de la peau, en particulier un fond de teint, un fard à joues ou à paupières, un mascara, un eye-liner, un stick anti-cernes, un vernis à ongles ou de maquillage des lèvres comme un rouge à lèvres. Elles peuvent également se présenter sous forme non colorée, contenant éventuellement des actifs cosmétiques ou dermatologiques. Elle peut alors être utilisée comme base de soin pour les lèvres (baumes à lèvres, protégeant les lèvres du froid et/ou du soleil et/ou du vent) ou base fixante à appliquer sur un rouge à lèvres classique.

La composition de l'invention peut également se présenter sous la forme d'une composition dermatologique ou cosmétique de traitement ou de soin de la peau (y compris le cuir chevelu), de fibres kératiniques (cheveux, cils, sourcils), des ongles ou des lèvres, ou sous forme d'une composition de protection solaire ou de bronzage artificiel, ou encore sous forme d'un produit nettoyant ou démaquillant de la peau ou des fibres kératiniques, un produit déodorant ou encore un produit parfumant.

Bien entendu la composition de l'invention doit être cosmétiquement ou dermatologiquement acceptable, à savoir non toxique et susceptible d'être appliquée sur la peau (y compris l'intérieur des paupières) ou les lèvres d'être humains.

De façon préférentielle, la composition de l'invention peut comprendre une matière colorante contenant notamment une phase particulaire, généralement présente à raison de 0 à 60 % du poids total de la composition, de préférence de 5 à 35 %, et qui peut comprendre des pigments et/ou des nacres et/ou des charges habituellement utilisés dans les compositions cosmétiques, ou encore des colorants solubles dans le milieu et notamment hydrosolubles ou liposolubles.

Par pigments, il faut comprendre des particules blanches ou colorées, minérales ou organiques, insolubles dans le milieu de la composition, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent à modifier la texture de la composition ainsi que l'effet de matité/brillance.

Les pigments peuvent être présents dans la composition à raison de 0 à 60 % du poids de la composition finale, et de préférence à raison de 4 à 25 %. Comme pigments miné-raux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium, aluminium et leurs mélanges.

Les nacres peuvent être présentes dans la composition à raison de 0 à 20 % du poids total de la composition, de préférence à un taux de l'ordre de 2 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 35 % du poids total de la composition, de préférence 5 à 15 %. On peut notamment citer le talc, le mica, la silice, les poudres de Nylon® (Orgasol® notamment de chez Atochem) et de polyéthylène, le Téflon®, l'amidon, le nitrure de bore, et les microbilles de résine de silicone (Tospearl® de Toshiba, par exemple) et leurs mélanges

Les colorants hydrosolubles sont notamment le jus de betterave, le bleu de méthylène et peuvent représenter de 0 à 6 % du poids total de la composition.

La composition selon l'invention peut être fabriquée à froid ou par chauffage d'un ou plusieurs organopolysiloxanes élastomériques sous forme de poudre dispersée dans de l'eau, ajout d'un ou plusieurs pigments, d'une ou plusieurs charges et/ou d'un ou plusieurs autres additifs, ajout éventuel de la phase grasse à l'état liquide (notamment portée à la température de fusion des cires la plus élevée), puis émulsification si nécessaire.

Elle peut aussi être obtenue par extrusion comme décrit dans la demande EP-A-667 146. Ce procédé consiste à malaxer la pâte (cires + huiles + additifs + pigments) pendant le refroidissement pour créer dans la masse des zones d'écrasement de la pâte à l'aide d'un broyeur à cylindres ou d'un extrudeur-mélangeur à vis. Ce procédé permet l'obtention d'une composition sous forme de pâte molle.

L'invention est illustrée plus an détail dans les exemples suivants. Les pourcentages sont donnés en poids.

### Exemple 1 :

### Réalisation d'un gel de protection solaire :

| | |
|---|---|
| ◆ Carbomer 980 | 0,3 % |
| ◆ TEA | 0,3 % |
| ◆ Silicone BY 29-119 | 15 % |
| ◆ TiO2 nanométrique traité hydrophile | 3 % |
| ◆ Méxoryl SX (*) | 0,5 % en Matière Active |
| ◆ Conservateur | qs |
| ◆ Eau | qsp 100 % |

| | |
|---|---|
| (*) l'acide benzène 1,4 [di(3-méthylidènecampho 10-sulfonique)] qui est un filtre hydrophile à large bande dans l'ultraviolet | |

### Résultat :

Obtention d'un gel très matifiant, d'une grande fraîcheur à l'application, de bonne tenue dans le temps et résistant bien à l'eau, contrairement aux produits de l'art antérieur.

### Préparation :

On ajoute l'organopolysiloxane à l'eau à température ambiante ensuite on ajoute le gélifiant, le neutralisant puis les TiO2, le Mexoryl SX et les conservateurs ; on mélange le tout sous agitation.

### Exemple 2 :

### Réalisation d'un fond de teint mat et frais résistant à l'eau :

| | |
|---|---|
| ◆ Silicone BY 29-122 | 70 % |
| ◆ Pigments (oxydes de fer) | 7 % |
| ◆ Talc | 10 % |
| ◆ Glycérine | 5 % |
| ◆ Conservateur | qs |
| ◆ Eau | qsp 100 % |

### Résultat :

Obtention d'un fond de teint frais, très mat, de bonne tenue dans le temps et résistant à l'eau.

### Préparation :

On prépare cette composition comme dans l'exemple 1.

On a réalisé un test comparatif de résistance à l'eau sur un fard à paupières à phase continue aqueuse, contenant ou non l'organopolysiloxane réticulé hydrophile. La résistance à l'eau a été mesurée sur un film de 50, 100, 150 et 300 µm déposé sur une plaque de verre qu'on a laissé séché à température ambiante pendant une heure. On a fait couler un filet d'eau en continu et mesurer le temps nécessaire pour commercer à dégrader le film.

### Exemple 3: Fard à paupière avec organopolysiloxane hydrophile

| | |
|---|---|
| ◆ Tréfil BY 29-119 | 32 % |
| ◆ Carbopol 980 | 0,58 % |
| ◆ TEA | 0,58 % |
| ◆ Nacre brune | 5 % |
| ◆ Talc | 5 % |
| ◆ Conservateur | 0,75 % |
| ◆ Eau | qsp 100 % |

| | | | | |
|---|---|---|---|---|
| Epaisseur du film étiré (µm) | 50 | 100 | 150 | 300 |
| Temps (s)* | 10 | 20 | 22 | 35 |

| | | | | |
|---|---|---|---|---|
| * Résistance à l'eau (après 1 h de séchage) | | | | |

### Contre exemple: Fard à paupière sans organopolysiloxane réticulé hydrophile

| | |
|---|---|
| ◆ Carbopol 980 | 0,58 % |
| ◆ TEA | 0,58 % |
| ◆ Nacre brune | 5 % |
| ◆ Talc | 5 % |
| ◆ Conservateur | 0,75 % |
| ◆ Eau | qsp 100 % |

| | | | | |
|---|---|---|---|---|
| Epaisseur du film étiré (µm) | 50 | 100 | 150 | 300 |
| Temps (s)** | 2 | 8 | 15 | 20 |

| | | | | |
|---|---|---|---|---|
| ** Résistance à l'eau (après 1 h de séchage) | | | | |

Le fard selon l'invention est remarquablement frais, doux, lisse et résistant à l'eau. Selon le test, on constate que le temps nécessaire à la dégradation du film en présence d'eau est supérieur pour la composition contenant l'organopolysiloxane à celui de la composition ne contenant pas cet organopolysiloxane.

## Revendications

1. Utilisation de particules d'organopolysiloxane solide élastomérique au moins partiellement réticulé en suspension dans une phase aqueuse, dans une composition cosmétique ou pour la fabrication d'une composition à application topique afin d'en augmenter sa résistance à l'eau.

2. Utilisation de particules d'organopolysiloxane solide élastomérique au moins partiellement réticulé en suspension dans une phase aqueuse, dans une composition cosmétique ou pour la fabrication d'une composition à application topique, résistante à l'eau.

3. Utilisation selon la revendication 1 ou 2, caractérisée en ce que la composition est une composition de maquillage ou de soin des matières kératiniques.

4. Utilisation selon l'une quelconque des revendications précédentes, caractérisée en ce que l'organopolysiloxane élastomérique est obtenu par réaction d'addition et de réticulation, en présence d'un catalyseur, d'au moins :
- un organopolysiloxane (i) ayant au moins deux groupes vinyliques en position α-ω de la chaîne siliconée par molécule ; et
- d'un organosiloxane (ii) ayant au moins un atome d'hydrogène lié à un atome de silicium par molécule.

5. Utilisation selon la revendication précédente, caractérisée en ce que l'organopolysiloxane (i) est choisi parmi les polydiméthylsiloxanes.

6. Utilisation selon l'une quelconque des revendications précédentes, caractérisée en ce que l'organopolysiloxane est un α-ω-diméthylvinyl polydiméthylsiloxane.

7. Utilisation selon l'une quelconque des revendications précédentes, caractérisée an ce que la suspension de particules d'organopolysiloxane est obtenue selon les étapes suivantes :
- (a) mélange d'organopolysiloxane (i) et d'organosiloxane (ii) ;
- (b) ajout de la phase aqueuse contenant un émulsifiant au mélange de l'étape (a) ;
- (c) émulsification de la phase aqueuse et dudit mélange ;
- (d) ajout d'eau chaude à l'émulsion de la phase (c) ; et
- (e) polymérisation de l'organopolysiloxane (i) et de l'organosiloxane (ii) en émulsion en présence d'un catalyseur de platine.

8. Utilisation selon la revendication précédente, caractérisée en ce que l'étape (c) est obtenue en présence d'un émulsifiant non-ionique.

9. Utilisation selon l'une quelconque des revendications précédentes, caractérisée an ce que les particules ont une taille allant de 0,1 à 500 µm et mieux de 3 à 200 µm.

10. Utilisation selon l'une quelconque des revendications précédentes, caractérisée an ce que les particules d'organopolysiloxane présentent une dureté inférieure ou égale à 80 et mieux inférieure à 65.

11. Utilisation selon l'une quelconque des revendications précédentes, caractérisée en ce qu'elle contient, an outre, une phase grasse.

12. Utilisation selon la revendication précédente, caractérisée en ce que la phase grasse contient au moins un corps gras choisi parmi les huiles volatiles ou non, les cires, les gommes et les corps gras pâteux, d'origine animale, végétale, minérale ou synthétique, et leurs mélanges.

13. Utilisation selon l'une des revendications précédentes, caractérisée en ce qu'elle comprend, en outre, un gélifiant de phase aqueuse.

14. Utilisation selon la revendication précédente, caractérisée en ce que le gélifiant de phase aqueuse est choisi parmi la gomme de xanthane, les argiles, les polyuréthanes associatifs, les épaississants cellulosiques, et les acides polyacryliques réticulés au moins partiellement neutralisés et leurs mélanges.

15. Utilisation selon l'une des revendications précédentes, caractérisée en ce que la composition comprend, en outre, une phase particulaire présente à raison de 0 à 60 % du poids total de la composition, de préférence 5 à 35 %.

16. Utilisation selon l'une des revendications précédentes, caractérisée en ce que la composition contient, en outre, au moins un actif cosmétique ou dermatologique.

17. Utilisation selon l'une des revendications précédentes, caractérisée en ce que la composition se présente sous forme d'une composition de fond de teint, de fard à joues ou à paupières, d'un produit anti-cernes, d'un rouge à lèvres, d'un eye-liner, d'un mascara, d'un vernis à ongles, d'une base de soin ou d'une base fixante pour les lèvres, d'un produit dermatologique ou de soin de la peau ou des fibres kératiniques, d'une composition de protection solaire ou de bronzage artificiel, d'un produit nettoyant de la peau ou des fibres kératiniques, un produit déodorant, un produit parfumant.

18. Utilisation selon l'une des revendications précédentes, caractérisée en ce que la composition contient, en outre, une matière colorante.

19. Utilisation selon l'une des revendications précédentes, caractérisée en ce que la composition contient, en outre, au moins un ingrédient choisi parmi les conservateurs, les antioxydants, les parfums, les gélifiants de phase grasse, les tensioactifs, leurs mélanges.

20. Procédé cosmétique pour augmenter la rémanence à l'eau d'une composition cosmétique, consistant à introduire dans la composition des particules d'un organopolysiloxane élastomérique au moins partiellement réticulé en suspension dans une phase aqueuse selon l'une des revendications 4 à 10.
